# EUROPÄISCHE PATENTSCHRIFT

(11) **EP 2 035 143 B1**
(45) Veröffentlichungstag und Bekanntmachung des Hinweises auf die Patenterteilung: **02.02.2011**
(21) Anmeldenummer: 07729737.2
(22) Anmeldetag: 31.05.2007
(51) Int. Cl.: B01L 3/00, C12Q 1/68

(54) **VERFAHREN ZUR ANALYSE VON AMPLIFIZIERTEN NUKLEINSÄUREN**
METHOD FOR ANALYSING AMPLIFIED NUCLEIC ACIDS
PROCÉDÉ D'ANALYSE D'ACIDES NUCLÉIQUES AMPLIFIÉS

(30) Priorität: 19.06.2006 DE 102006028101
(43) Veröffentlichungstag der Anmeldung: 18.03.2009
(73) Patentinhaber: SIEMENS AKTIENGESELLSCHAFT, 80333 München (DE)
(72) Erfinder: GUMBRECHT, Walter, 91074 Herzogenaurach (DE); PAULICKA, Peter, 91056 Erlangen (DE); STANZEL, Manfred, 91056 Erlangen (DE)
(86) Internationale Anmeldenummer: PCT/EP2007/055335
(87) Internationale Veröffentlichungsnummer: WO 2007/147712

(56) Entgegenhaltungen:
- DE-A1- 10 111 457
- US-A1- 2004 007 275
- ROBIN HUI LIU ET AL: "SELF-CONTAINED, FULLY INTEGRATED BIOCHIP FOR SAMPLE PREPARATION, POLYMERASE CHAIN REACTION AMPLIFICATION, AND DNA MICROARRAY DETECTION" ANALYTICAL CHEMISTRY, AMERICAN CHEMICAL SOCIETY. COLUMBUS, US, Bd. 76, Nr. 7, 1. April 2004 (2004-04-01), Seiten 1824-1831, XP001196720 ISSN: 0003-2700
- LIU ROBIN HUI ET AL: "Integrated microfluidic biochips for DNA microarray analysis." EXPERT REVIEW OF MOLECULAR DIAGNOSTICS MAR 2006, Bd. 6, Nr. 2, März 2006 (2006-03), Seiten 253-261, XP009087908 ISSN: 1744-8352
- ZHANG ET AL: "PCR microfluidic devices for DNA amplification" BIOTECHNOLOGY ADVANCES, ELSEVIER PUBLISHING, BARKING, GB, Bd. 24, Nr. 3, Mai 2006 (2006-05), Seiten 243-284, XP005362290 ISSN: 0734-9750

## Beschreibung

Die Erfindung betrifft ein Verfahren zur Analyse von amplifizierten Nukleinsäuren in einer mikrofluidischen Vorrichtung. Die Erfindung betrifft ferner eine Anordnung zur Durchführung eines solchen Verfahrens.

### Stand der Technik

Die DNA-Analyse durch Hybridisierung ist ein in der Molekularbiologie bekanntes Verfahren (vgl. "Gentechnische Methoden", G.Gassen und G.Schrimpf, Spektrum Akademischer Verlag Heidelberg, 1999, Seite 243 bis 261). Diese Technik spielt beim Nachweis spezifischer Nukleinsäuren eine wichtige Rolle, z.B. in der Molekulardiagnostik von Einzelpunktmutationen (single nucleotide polymorphism, SNP). Hierbei wird ein Sonden-Oligonukleotid, das aus einer Sequenz von z.B. ca. 20 Nukleotiden besteht, verwendet, um an Nukleinsäuren zu binden, die sich nur in einem einzigen Nukleotid unterscheiden. Es wird angemerkt, dass im vorliegenden Kontext der Begriff "Nukleinsäuren" eine Nukleinsäuresequenz, z.B. eine DNA-Sequenz oder RNA-Sequenz, umfassen soll. Bei geeigneter Wahl der Hybridisierungsbedingungen (insbesondere Temperatur und Salzkonzentration), bindet das Sonden-Oligonukleotid selektiv die nicht mutierte Variante der Nukleinsäure, während die Nukleinsäurevariante, welche die Einzelpunktmutation aufweist, nicht bindet bzw. nur schwach bindet. Dadurch ist ein Nachweis von Einzelpunktmutationen möglich. Aufgrund der geringen bindungsenergetischen Unterschiede zwischen der Variante ohne Mutation (d.h. dem Wildtyp) und der Mutante müssen die Reaktionsbedingungen bezüglich Temperatur sowie Zusammensetzung und Salzkonzentration der Reaktionslösung exakten Vorgaben genügen.

Da die entsprechenden Nukleinsäuren im Probenmaterial (z.B. Blut) meist nicht in ausreichender Menge bzw. Konzentration zur Verfügung stehen, ist es notwendig, die zu untersuchenden Nukleinsäuren zu amplifizieren. Diese Amplifikation kann durch verschiedene, in der Molekularbiologie bekannte Verfahren sequenzspezifisch erfolgen, z.B. durch SDA (strand displacement amplification), beschrieben in Walker, GT, et al., "Strand Displacement Amplification, an isothermal, invitro DNA Amplification Technique", Nucleic Acids Research, 1992, 20, 1961 bis 96; durch TMA (transcription mediated amplification), beschrieben in www.gen-probe.com/sci_tec/tea.htm; oder durch Polymerase-Kettenreaktion (polymerase chain reaction, PCR), beschrieben u.a. in US 4 683 195. Ein Problem hierbei ist, dass die Zusammensetzung der Reaktionslösung der Amplifikationsreaktion, und somit das "Amplifikationsrohprodukt", nicht die zur Hybridisierung erforderliche Zusammensetzung und insbesondere Salzkonzentrationen aufweist. Zur molekularen Diagnostik kann es außerdem notwendig sein, die gebildeten Hybride in einem Folgeprozess (Aufschmelzen) z.B. durch Temperaturerhöhung selektiv zu trennen. Um Hybridisierungsprozesse mit hoher Ausbeute zu ermöglichen, ist u.a. eine hohe Konzentration an monovalenten Kationen (z.B. Na⁺- Ionen) erforderlich. Monovalente Kationen begünstigen die Bildung der Doppelhelix-Struktur bei der Hybridisierungsreaktion.

Die Reaktionsansätze der Amplifikationsreaktionen, z.B. für eine PCR-Reaktion, enthalten jedoch eine niedrige Konzentration an monovalenten Kationen. Ferner weisen PCR-Reaktionspuffer eine relativ hohe Konzentration (einige mM) an Mg²⁺-Ionen auf, welche sich bei der Hybridisierung an Detektions-Sonden nachteilig auf die Bindung von Sonden und komplementären Strängen zu vollständigen Hybriden auswirken, eine Verlängerung der Sonden durch Polymerase-Aktivität bewirken können und bei einem anschließenden Aufschmelzvorgang eine Stabilisierung von Doppelsträngen bewirken und das Aufschmelzen erschweren, was zu "verwaschenen" Schmelzkurven bei hohen Temperaturen führt.

Gemäß dem Stand der Technik werden Amplifikationsprodukte deshalb vor der Hybridisierungsreaktion aufgereinigt, dabei werden alle, eine Hybridisierungsreaktion störenden Komponenten (u.a. Polymerase, Primer, Nukleotide, Salze) entfernt und die Konzentration an Na⁺-Ionen erhöht. Dieser Aufreinigungsprozess ist relativ aufwendig und erfolgt üblicherweise durch unspezifische Bindung der Nukleinsäuren an eine feste Phase (durch sogenannte Reinigungssäulen), Waschen des Amplifikationsproduktes auf der Säule und Lösen von der festen Phase oder durch Phenol/Chloroformextraktion oder ähnliche Verfahren. Insbesondere bei der Durchführung von Nukleinsäureanalysen in mikrofluidischen Vorrichtungen, wobei alle Reaktionsvorgänge integriert und auf kleinem Raum ablaufen, kommen die im Stand der Technik üblichen Aufreinigungsverfahren nicht in Frage, da ihre Realisierung unter diesen Umständen zu aufwändig ist.

Liu et al (Anal. Chem. 2004, 76, 1824-1831) offenbart ein Verfahren zur Analyse von Nukleinsäuren in einer mikrofluidischen Vorrichtung umfassend die Schritte Amplifizieren von Nukleinsäuren in einer ersten Kammer der mikrofluidischen Vorrichtung, in Kontakt Bringen der amplifizierten Nukleinsäuren mit einem Hybridisierungspuffer in einer zweiten Kammer in der mikrofluidischen Vorrichtung und Hybridisieren der amplifizierten Nukleinsäuren an mindestens ein Sonden-Oligonukleotid.

### Aufgabenstellung

Aufgabe der vorliegenden Erfindung ist es, ein einfaches und kostengünstiges Verfahren bereitzustellen, welches die effiziente Aufbereitung von amplifizierten Nukleinsäuren für weitere Verfahrensschritte ermöglicht, welches keine zusätzlichen Bindungs- oder Waschschritte erfordert und sich mit einem einfachen Fluidik-Konzept realisieren lässt.

### Beschreibung der Erfindung

Allgemein ausgedrückt liegt der Erfindungsgedanke darin, eine Probe mit Nukleinsäuren zu amplifizieren und die Probe, welche das Amplifikationsrohprodukt enthält, mit einem geeigneten Zusatz zu versetzen, um das Amplifikationsrohprodukt für weitere Verfahrensschritte, z.B. weitere Analyseschritte, aufzubereiten. Durch Zugabe des Zusatzes wird das Erfordernis, die amplifizierten Nukleinsäuren aufzureinigen, umgangen. Dieses Verfahren ist besonders für den Einsatz in mikrofluidischen Vorrichtungen geeignet, in welchen unkomplizierte Verfahrensabläufe mit einem einfachen Fluidik-Konzept bevorzugt sind. Erfindungsgemäß weist der Zusatz monovalente Kationen und ein Mg²⁺-ionenbindendes Agens auf. Ein derartiger Zusatz ist insbesondere zur Aufbereitung von amplifizierten Nukleinsäuren für eine folgende Hybridisierung an Sonden-Oligonukleotide geeignet.

Erfindungsgemäß wird die Aufgabe insbesondere durch das Verfahren gemäß Patentanspruch 1 gelöst. Vorteilhafte Weiterbildungen sind in den abhängigen Verfahrensansprüchen angegeben. Eine zugehörige Anordnung zur Durchführung des erfindungsgemäßen Verfahrens ist Gegenstand des Patentanspruchs 16. Weiterbildungen dieser Anordnung sind in den abhängigen Sachansprüchen angegeben.

Gemäß der vorliegenden Erfindung wird insbesondere ein Verfahren zur Analyse von amplifizierten Nukleinsäuren in einer mikrofluidischen Vorrichtung bereitgestellt, welches die folgenden Schritte aufweist:
a) Amplifizieren von Nukleinsäuren in einer ersten Kammer in der mikrofluidischen Vorrichtung;
b) In Kontakt Bringen der amplifizierten Nukleinsäuren mit einem Zusatz geeignet zur Aufbereitung von amplifizierten Nukleinsäuren für eine folgende Hybridisierung an Sonden-Oligonukleotide, aufweisend:
   i.) monovalente Kationen und
   ii.) ein Mg²⁺-ionenbindendes Agens,
   wobei der Zusatz in einer zweiten Kammer in der mikrofluidischen Vorrichtung als Trockenreagenz bevorrakt vorgesehen ist und als Trockenreagenz hervorragt;
c) Hybridisieren der amplifizierten Nukleinsäuren an mindestens ein Sonden-Oligonukleotid, wobei zur Hybridisierungsreaktion eine Mischung aus Amplifikationsrohprodukt und Zusatz verwendet wird.

Der Ausdruck "mikrofluidisch" bezeichnet Verfahren, welche die Handhabung von Fluiden mit Volumina im Mikroliter-Bereich umfassen. Die mikrofluidische Vorrichtung ist vorzugsweise als Cartridge ausgebildet, d.h. als ein Flachgebilde, welches die Form einer Karte aufweist, mit darin ausgebildeten Vertiefungen, welche Kanäle und Kammern bzw. Kavitäten bilden, durch welche Flüssigkeiten gemäß vorbestimmten Reaktionsabläufen oder Schemata bewegt werden können.

Monovalente Kationen umfassen z.B. Li⁺, Na⁺, Ka⁺, und liegen in dem erfindungsgemäßen Zusatz vorzugsweise in Form von Na⁺-Ionen vor. Unter einem Mg²⁺-Ionen-bindendem Agens sind sämtliche Stoffe zu verstehen, welche Mg²⁺ - Ionen binden, insbesondere Komplexbildner, z.B. Chelat-Komplexbildner wie EGTA oder EDTA. Vorzugsweise weist der im erfindungsgemäßen Verfahren verwendete Zusatz EDTA auf. Ferner weist der Zusatz bevorzugt ein Bindemittel auf, z.B. Polyvinylpyrrolidon. Weitere Hilfsstoffe, z.B. Puffersubstanzen, oberflächenaktive Stoffe o.ä., können ebenfalls vorgesehen sein.

Der Zusatz ist in der zweiten Kammer als Trockenreagenz vorgesehen und lagerstabil bevorratet.

Bevorzugt sind die Sonden-Oligonukleotide als Mikroarray auf einem Träger in der mikrofluidischen Vorrichtung immobilisiert.

Gemäß einer ersten Ausführungsform des Verfahrens der vorliegenden Erfindung wird der Zusatz von der zweiten Kammer in die erste Kammer (die Amplifikationskammer) überführt, um den Zusatz mit den amplifizierten Nukleinsäuren in Kontakt zu bringen. Falls der Zusatz bei dieser Ausführungsform des erfindungsgemäßen Verfahrens in der zweiten Kammer als Trockenreagenz vorgesehen ist, ist es zweckmäßig, den Zusatz mit einem Lösungsmittel, z.B. Wasser, in Lösung zu bringen. Der gelöste Zusatz kann dann von der zweiten Kammer in die erste Kammer überführt werden, um sich dort mit dem Amplifikationsrohprodukt zu vermischen.

Gemäß einer zweiten Ausführungsform der vorliegenden Erfindung werden nach erfolgter Amplifikation die amplifizierten Nukleinsäuren in der Reaktionslösung von der ersten Kammer in die zweite Kammer überführt und dann als Gemisch mit dem Zusatz zu den Sonden-Oligonukleotiden geleitet. Falls der Zusatz als Trockenreagenz in der zweiten Kammer vorgesehen ist, kann er direkt durch die in die zweite Kammer gepumpte Reaktionslösung mit dem Amplifikationsrohprodukt in Lösung gebracht werden.

Erfindungsgemäß ist es bevorzugt, dass die Amplifikation der Nukleinsäuren durch PCR-Reaktion erfolgt.

Gemäß einem weiteren Aspekt der vorliegenden Erfindung erfolgt dann bevorzugt eine Detektion der an die Sonden-Oligonukleotide hybridisierten, amplifizierten Nukleinsäuren. Diese Detektion kann beispielsweise unter Ausnutzung eines Labels (einer Markierung) der amplifizierten Nukleinsäuren erfolgen. Das Label kann ein optisches Label sein, es kann beispielsweise auch ein enzymatisches Label sein. Durch ein enzymatisches Label kann eine enzymatische Reaktion katalysiert werden, die z.B. optisch oder elektrochemisch detektiert werden kann. Erfindungsgemäß wird vorzugsweise eine elektrochemische Detektion durchgeführt, die besonders bevorzugt eine mittels Redox-Cycling verstärkte Strommessung umfasst.

Insbesondere betrifft die Erfindung ferner eine Anordnung zur Durchführung des erfindungsgemäßen Verfahrens, welches in einer mikrofluidischen Vorrichtung vorgesehen ist und eine erste Kammer, welche für die Amplifikation von Nukleinsäuren ausgelegt ist, und eine zweite Kammer, in welcher der oben beschriebene Zusatz lagerstabil bevorratet ist, wobei die zweite Kammer über eine Verbindung mit der ersten Kammer in Fluidkommunikation verbindbar ist. Vorzugsweise umfasst die Anordnung eine Mikroarray-Anordnung, die auf einem Träger immobilisierte Sonden-Oligonukleotide aufweist.

Die Verbindung zwischen der ersten und zweiten Kammer kann in Form einer Leitung oder eines Kanals ausgebildet sein, und kann vorzugsweise selektiv geöffnet und geschlossen werden kann, z.B. durch ein Ventil, so dass selektiv Fluid von der ersten in die zweite Kammer, oder von der zweiten in die erste Kammer überführt werden kann. Ferner können Mittel vorgesehen sein, um ein Lösungsmittel in die zweite Kammer einzubringen, z.B. in Form eines Zuflusskanals zu der zweiten Kammer.

Vorzugsweise sind der Mikroarray-Anordnung Mittel zur Detektion von hybridisierten Nukleinsäuren zugeordnet, welche beispielsweise eine optische oder elektrochemische Detektion ermöglichen. Besonders bevorzugt sind Mittel zur elektrochemischen Detektion, die zur Messung von Strömen und/oder Potenzialen ausgebildet sind. Die Mittel zur optischen Detektion können z.B. einen transparenten Bereich der Vorrichtung umfassen, durch welchen z.B. optische Absorption oder Fluoreszenzanregung und- detektion auslesbar ist. Die Mittel zur elektrochemischen Detektion ermöglichen bevorzugt die Messung von Potentialen und/oder Strömen und können ein Elektrodensystem umfassen, auf welches die Sonden-Oligonukleotide an jedem Detektions-Spot immobilisiert (aufgespottet) sind, wie beispielsweise in den Schriften DE 101 26 341 A1 oder DE 100 58 397 A1 beschrieben. DE 101 11 457 offenbart die Verwendung von in mikrofluidalen Vorrichtungenals Trockenreagenzien vorliegenden Reagenzien.

Weiterhin können zur Bevorratung und/oder Weiterleitung von Reagenzien für die Detektion, z.B. Enzym oder Enzymsubstrat, in der mikrofluidischen Vorrichtung entsprechende Mittel, z.B. in Form entsprechender Kammern und/oder Kanäle, vorgesehen sein.

Der ersten Kammer (also der Amplifikationskammer) sind vorzugsweise Mittel zur Wärmezufuhr und/oder Wärmeabfuhr zugeordnet. Diese Mittel können einen Bereich mit erhöhter Wärmeleitfähigkeit in der mikrofluidischen Vorrichtung umfassen, der beispielsweise dadurch realisiert sein kann, dass die mikrofluidische Vorrichtung in diesem Bereich besonders dünnwandig ausgebildet ist. Es ist aber auch denkbar, dass in der mikrofluidischen Vorrichtung selber ein Wärme erzeugendes oder abführendes Element vorgesehen ist.

### Ausführungsbeispiel

Weitere Merkmale und Vorteile der vorliegenden Erfindung ergeben sich aus der folgenden Figurenbeschreibung im Zusammenhang mit den Ausführungsbeispielen und anhand der angehängten Zeichnungen, welche lediglich beispielhaft und veranschaulichend sind.

Es zeigen:
- Figur 1: eine diagrammartige Darstellung des erfin- dungsgemäßen Verfahrens;
- Figur 2: eine schematische Darstellung einer Anordnung zur Durchführung des Verfahrens der Erfindung gemäß einer ersten Ausführungsform;
- Figur 3: eine schematische Darstellung einer Anordnung zur Durchführung des Verfahrens der Erfindung gemäß einer zweiten Ausführungsform;
- Figur 4: einen Ausschnitt einer schematischen Darstel- lung einer Cartridge mit der Anordnung gemäß Figur 2;
- Figur 5: eine vergleichende Darstellung von Schmelzkur- ven von hybridisierten Nukleinsäuren ohne Ver- wendung des erfindungsgemäßen Verfahrens; und
- Figur 6: eine vergleichende Darstellung von Schmelzkur- ven von hybridisierten Nukleinsäuren unter Verwendung des erfindungsgemäßen Verfahrens.

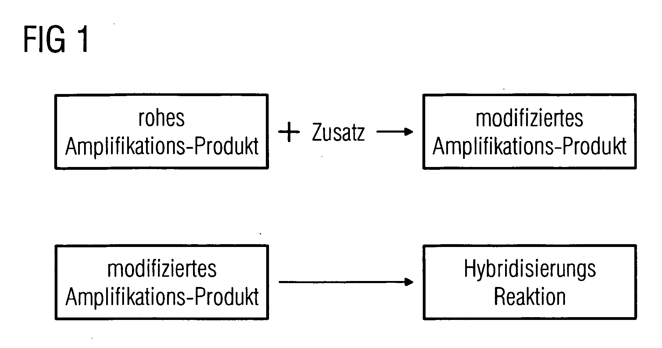

In Figur 1 ist schematisch der Grundgedanke des erfindungsgemäßen Verfahrens zur Analyse von Nukleinsäuren in einer mikrofluidischen Vorrichtung dargestellt. Gemäß dem Stand der Technik ist es bekannt, Amplifikationsrohprodukte vor der Hybridisierung aufzureinigen. Dies stellt ein subtraktives Verfahren dar, d.h. aus der das Amplifikationsrohprodukt enthaltenden Lösung werden störende Komponenten (Polymerase, Primer, Nukleotide) entfernt. Dem gegenüber ist das erfindungsgemäße Verfahren ein additives Verfahren, d.h. dem Amplifikationsrohprodukt wird ein Zusatz zugefügt, welcher eine verbesserte Hybridisierungsreaktion, bzw. eine nachfolgende verbesserte, selektive Auftrennung der Hybride ermöglicht. Dabei spielt es keine Rolle, ob der Zusatz zu dem Amplifikationsrohprodukt zugefügt wird oder das Amplifikationsrohprodukt zum Zusatz zugefügt wird, entscheidend ist vor allem, dass zur Hybridisierungsreaktion eine Mischung aus Amplifikationsrohprodukt und Zusatz verwendet wird.

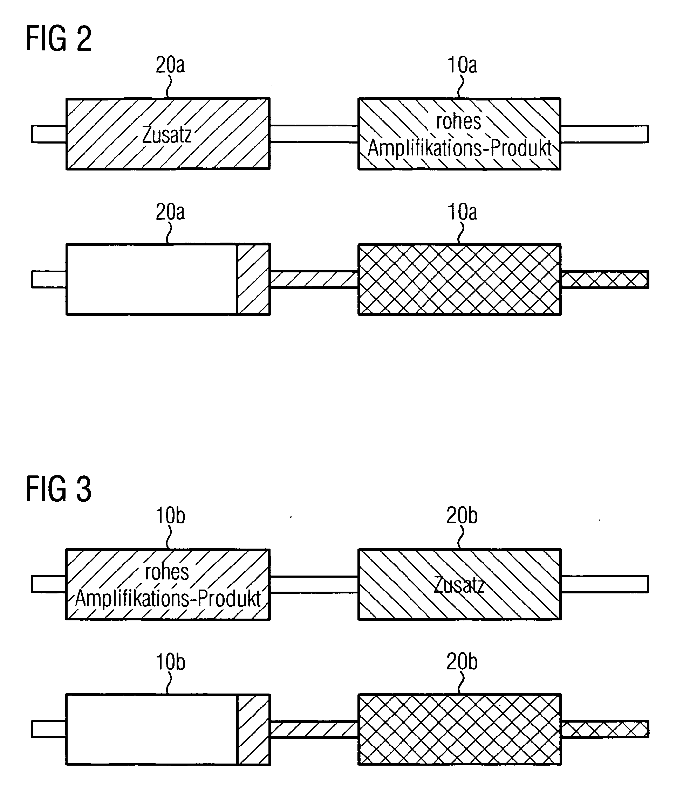

Figur 2 zeigt eine Anordnung zur Durchführung des erfindungsgemäßen Verfahrens gemäß einer ersten Ausführungsform mit einer ersten Kammer 10a und zweiten Kammer 20a. Nach Ablauf der Amplifikationsreaktion wird der in Lösung befindliche Zusatz von der zweiten Kammer 20a (der Zusatzkammer) in die erste Kammer 10a (die Amplifikations-Reaktions-Kammer) gepumpt. Falls der Zusatz in der zweiten Kammer als Trockenreagenz vorgesehen ist, wird zunächst ein Lösungsmittel (Wasser) in die zweite Kammer 20a gepumpt, um das Trockenreagenz zu lösen, dann wird die Lösung in die erste Kammer 10a (Amplifikations-Reaktions-Kammer) überführt.

Figur 3 zeigt eine Anordnung zur Durchführung des erfindungsgemäßen Verfahrens gemäß einer zweiten Ausführungsform mit einer ersten Kammer 10b und zweiten Kammer 20b. Hierbei wird in umgekehrter Weise zu der oben beschriebenen Verfahrensführung vorgegangen: nach der Amplifikationsreaktion wird das Amplifikationsrohprodukt aus der ersten Kammer 10b (der Amplifikations-Reaktions-Kammer) in die zweite Kammer 20b (die Zusatzkammer) geleitet und dort mit dem Zusatz durchmischt. Falls der Zusatz als Trockenreagenz vorliegt, wird das Amplifikationsrohprodukt über das Trockenreagenz gepumpt und löst dieses auf.

Bei beiden Ausführungsformen wird dann das Gemisch aus Amplifikationsrohprodukt und dem Zusatz zum Mikroarray zur Hybridisierung überführt.

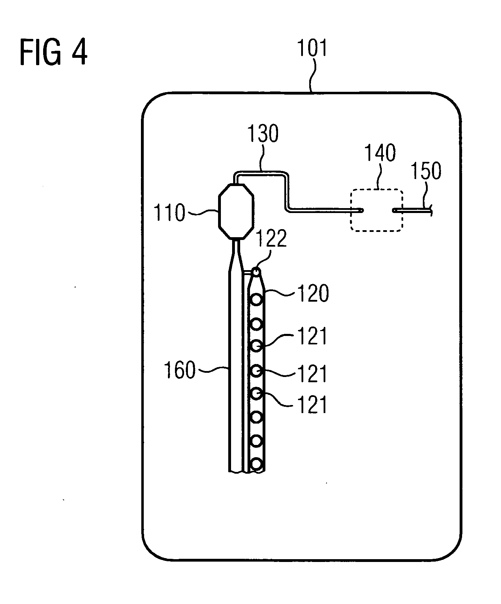

Figur 4 zeigt eine Anordnung gemäß der in Figur 2 gezeigten Ausführungsform. Figur 4 zeigt eine Cartridge 101, welche aus einem Kunststoffmaterial gemacht ist. In der Cartridge 101 sind Kanäle 130, 150, 160, Kammern 110, 120, 140 und Vertiefungen 121 vorgesehen. Die Kunststoff-Cartridge kann einfach mit nach oben offenen Kanälen, Vertiefungen und Kammern ausgebildet sein und nach aufspotten der Reagenzien mit einer Folie abgedeckt werden, wodurch die in die Oberfläche der Cartridge eingearbeiteten Kanäle, Vertiefungen und Kammern abgeschlossen werden. In der ersten Kammer 110, die über den Zufluss-Kanal 160 befüllbar ist, werden Nukleinsäuren beispielsweise durch PCR-Reaktion amplifiziert. Dies geschieht, indem die Cartridge in eine entsprechende Vorrichtung gesteckt wird, so dass die Kammer 110, z.B. durch in der Vorrichtung befindliche Peltier-Elemente, beheizbar und/oder kühlbar ist. Der Zusatz, aufweisend EDTA und Natriumchlorid, liegt als Trockenreagenz in den Vertiefungen 121 in der als Kanal ausgebildeten zweiten Kammer 120 vor. An seinem einen Ende ist die Kammer 120 durch ein Ventil 122 verschließbar. Dies kann beispielsweise als einfaches Quetschventil ausgebildet sein. Nach erfolgter PCR-Reaktion wird in die Kammer 120 Wasser gepumpt, und dadurch wird der als Trockenreagenz bevorratete Zusatz gelöst. Anschließend wird das Ventil 122 geöffnet und der gelöste Zusatz kann nun in die Amplifikationskammer 110 gepumpt werden. Dort durchmischt sich der gelöste Zusatz mit dem Amplifikationsrohprodukt, und die Mischung wird dann über den Kanal 130 in die Hybridisierungskammer 140 gepumpt, in welcher ein Mikroarray mit auf einem Träger immobilisierten Sonden-Oligonukleotiden vorgesehen ist. Durch geeignete Wahl der Fluidik, z.B. Geometrie oder Fließgeschwindigkeiten, kann realisiert werden, dass zu Beginn des Pumpvorganges zunächst unverändertes Amplifikationsprodukt gefördert wird und erst bei weiterem Pumpen zunehmend Zusatzstoffe beigemischt werden. Somit kann ein gegebenenfalls vorteilhafter, ansteigender Konzentrations-Gradient an Zusatzstoffen in die Hybridisierungskammer gepumpt werden. In dieser Kammer kann nun die Hybridisierungsreaktion stattfinden. Überschüssige Lösung wird über den Abflusskanal 150 einem Abfallbehälter (waste) zugeführt. Auf der Cartridge können weitere Kammern und Kanäle vorgesehen sein (nicht gezeigt), um z.B. Reagenzien für die Detektion der gebundenen Nukleinsäuren bereit zu halten, wie etwa Enzym oder Enzymsubstrat.

### Beispiel

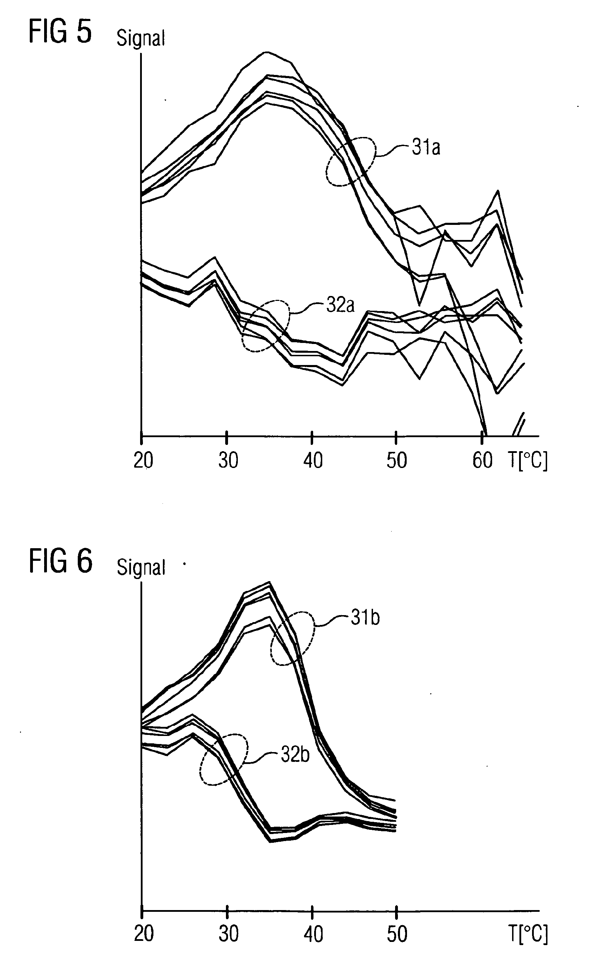

Für die Aufnahme der in Figuren 5 und 6 gezeigten Kurven wurde eine Spritzguss-Kunststoffkarte der in Figur 4 gezeigten Art verwendet, in welcher in den Vertiefungen 121 Trockenreagenz aufgespottet war. Um das erfindungsgemäße Verfahren zu evaluieren, wurde das Gen Faktor V Wildtyp (FcV-Wildtyp) und die Einzelpunktmutation Faktor V Leiden (FcV-Leiden) durch PCR amplifiziert, um ein 168 bp großes PCR-Produkt zu erhalten. Das Genprodukt des Faktor V Gens ist ein Protein der Blutgerinnungskaskade, die Mutation ist beschrieben in Bertima et al., Nature, 1994; 369(6475):64-7.

Die DNA- Probe wird in die erste Kammer (Amplifikationskammer) 110 eingebracht und die PCR-Reaktion wird durchgeführt. Der Kanal 120 mit den Vertiefungen 121 wird mit Wasser gefüllt, und dadurch geht das Trockenreagenz in Lösung. Der als Trockenreagenz aufgespottete Zusatz ist so bemessen, dass bei Zugabe von ca. 25 µl Wasser eine Lösung mit 0,23M NaCl, 0,1M EDTA resultiert. Dadurch ist eine ausreichende Konzentration von monovalenten Kationen sowie die Minimierung der Konzentration an freien Mg²⁺ durch Komplexierung gewährleistet. Ferner ist dem Zusatz eine Puffersubstanz zugesetzt, so dass die Lösung auf einen pH-Wert von pH=8 eingestellt ist. Nach Beendigung der PCR-Reaktion wird durch Einströmen des gelösten Zusatzes in die erste Kammer 110 das PCR-Rohprodukt (Amplifikationsrohprodukt) mit dem Zusatz gemischt, und die Mischung wird in die Kammer 140 weitergeleitet, in welcher sich eine Mikroarray-Anordnung befindet. Durch Verwendung biotinylierter Primer bei der PCR-Reaktion werden biotinylierte PCR-Produkte erhalten. Auf der Mikroarray-Anordnung sind Sonden-Oligonukleotide gespottet, über welche zwischen FcV-Wildtyp und FcV-Leiden diskriminiert werden kann. Die Sonden-Oligonukleotide sind so gewählt, dass einige Spots Sonden-Oligonukleotide tragen, die perfekt zu FcV-Wildtyp passen, während andere Spots Sonden-Oligonukleotide tragen, die perfekt zu FcV-Leiden passen. Befinden sich im PCR-Produkt z.B. FcV-Wildtyp Sequenzen, dann bilden sie mit den Wildtyp-Sonden einen "perfect match" (eine vollkommene Paarung von Strang und Gegenstrang), mit den FcV-Leiden Sonden jedoch einen "single-base-mismatch" (eine einzelne Basenfehlpaarung), der eine geringere Bindungsstärke aufweist. Die an Sondenmoleküle gebundenen PCR-Produkte werden mit einer Waschlösung überspült, welche ein Streptavidin-konjugiertes Enzym (alkalische Phosphathase) enthält. Das Enzym bindet an die biotinylierten PCR-Produkte, welche auf der Mikroarray-Anordnung gebunden sind. Nun wird die Mikroarray-Anordnung mit einer Substratlösung überspült, welche p-Aminophenylphosphat enthält. Das p-Aminophenylphosphat wird von der alkalischen Phosphathase zu p-Aminophenol umgesetzt und das gebildete p-Aminophenol wird in einer Redoxreaktion an Elektroden der Mikroarray-Anordnung zu Chinonimin oxidiert und das Redoxpaar p-Aminophenol/Chinonimin wird zyklisiert, was an den Elektroden zu einem messbaren Stromanstieg führt. Dieser Stromanstieg (dI/dT) ist proportional zu der Menge an gebundenem PCR-Produkt. Nun wird schrittweise über einen Temperaturbereich von ca. 20°C bis ca. 60°C die Temperatur erhöht, was bei höheren Temperaturen (ab ca. 25°C) zu einem sukzessiven Aufschmelzen der Hybride führt, wobei die PCR-Produkte mit Einzelpunktmutationen, die im Hybrid eine Fehlpaarung (mismatch) aufweisen, deutlich rascher aufschmelzen als die "perfect-match"-Hybride, im Beispiel die Wildtyp-Hybride. Dadurch ergibt sich ein detektierbarer Signalunterschied zwischen dem Wildtyp (perfect match) und der Einzelpunktmutante (single base mismatch). In den in Figur 5 und 6 gezeigten Kurven 31a, 31b, 32a, 32b ist die Zusammenfassung mehrerer Experimente dargestellt. Dargestellt ist die Signalstärke in Abhängigkeit der Temperatur. Die Kurven 31 a, 31b zeigen die Schmelzkurven für FcV-Wildtyp und die Kurven 32a, 32b für FcV -Leiden. Es lässt sich ohne weiteres erkennen, dass bei Verwendung des erfindungsgemäßen Verfahrens mit dem zugesetzten Zusatz die Schmelzkurven 31b, 32b (Fig. 6) wesentlich besser reproduzierbar sind (d.h. für FcV-Wildtyp und FcV-Leiden jeweils enger beieinander liegen) und deutlichere Signalunterschiede resultieren als ohne Verwendung des erfindungsgemäßen Verfahrens 31a, 32a (Fig. 5). Vor allem bei höheren Temperaturen (T>35°C) schmelzen die Hybride wesentlich sauberer auf. Eine vorteilhafte Eigenschaft dieses Verfahrens könnte sein, dass während der Hybridisierung die Konzentration an NaCl und EDTA in der Kammer 140 (der Detektionskammer) ansteigt.

Es wird betont, dass das beschriebene Ausführungsbeispiel lediglich beispielhaft ist, und vielerlei Variationen bezüglich der Art und Konzentration des Zusatzes, der Art der Detektion und der Reaktionsführung denkbar sind.

## Patentansprüche

1. Verfahren zur Analyse von Nukleinsäuren in einer mikrofluidischen Vorrichtung, aufweisend die folgenden Schritte:
a) Amplifizieren von Nukleinsäuren in einer ersten Kammer in der mikrofluidischen Vorrichtung;
b) in Kontakt Bringen der amplifizierten Nukleinsäuren mit einem Zusatz, geeignet zur Aufbereitung von amplifizierten Nukleinsäuren für eine folgende Hybridisierung an Sonden-Oligonukleotide aufweisend:
i) monovalente Kationen und
ii) ein Mg²⁺ Ionen-bindendes Agens,
wobei der Zusatz in einer zweiten Kammer in der mikrofluidischen Vorrichtung als Trockenreagenz bevorratet vorgesehen ist; und
c) Hybridisieren der amplifizierten Nukleinsäuren an mindestens ein Sonden-Oligonukleotid, wobei zur Hybridisierungsreaktion eine Mischung aus Amplifikationsrohprodukt und Zusatz verwendet wird.

2. Verfahren nach Anspruch 1, wobei die monovalenten Kationen in Form von Na⁺ Ionen vorgesehen sind.

3. Verfahren nach Anspruch 1 oder 2, wobei das Mg²⁺ Ionen -bindende Agens EDTA ist.

4. Verfahren nach Anspruch 1, wobei vor in Kontakt Bringen des Zusatzes mit den amplifizierten Nukleinsäuren ein Lösungsmittel in die zweite Kammer eingebracht wird.

5. Verfahren nach Anspruch 4, wobei der Zusatz in gelöster Form von der zweiten in die erste Kammer überführt wird, um den Zusatz mit den amplifizierten Nukleinsäuren in Kontakt zu bringen.

6. Verfahren nach einem der Ansprüche 1 bis 3, wobei die amplifizierten Nukleinsäuren von der ersten in die zweite Kammer überführt werden, um den Zusatz mit den amplifizierten Nukleinsäuren in Kontakt zu bringen.

7. Verfahren nach Anspruch 6, wobei die amplifizierten Nukleinsäuren in Lösung als Amplifikationsrohprodukt in die zweite Kammer überführt werden und dann als Gemisch mit dem Zusatz zu dem mindestens einen Sonden-Oligonukleotid geleitet werden.

8. Verfahren nach einem der vorangehenden Ansprüche, wobei die Amplifikation der Nukleinsäuren mittels PCR Reaktion erfolgt.

9. Verfahren nach einem der vorangehenden Ansprüche, wobei das mindestens eine Sonden-Oligonukleotid in Form einer Mikroarray-Anordnung auf einem Träger immobilisiert ist.

10. Verfahren nach einem der vorangehenden Ansprüche, wobei eine Detektion der an das hybridisierten, amplifizierten Nukleinsäuren erfolgt.

11. Verfahren nach Anspruch 10, wobei die Detektion unter Ausnutzung eines Labels (einer Markierung) der amplifizierten Nukleinsäuren erfolgt.

12. Verfahren nach Anspruch 11, wobei das Label ein optisches oder ein enzymatisches Label ist.

13. Verfahren nach Anspruch 12, wobei das Label eine enzymatische Reaktion katalysiert, die optisch oder elektrochemisch detektierbar ist.

14. Verfahren nach Anspruch 13, wobei es sich bei der elektrochemischen Detektion um eine mittels Redoxcycling verstärkte Strommessung handelt.

15. Verfahren nach einem der vorangehenden Ansprüche, wobei der Zusatz ein Bindemittel aufweist.

16. Anordnung zur Durchführung des Verfahrens gemäß einem der Ansprüche 1 bis 15, welche in einer mikrofluidischen Vorrichtung vorgesehen ist, aufweisend:
a) eine erste Kammer, welche für die Amplifikation von Nukleinsäuren ausgelegt ist , und
b) eine zweite Kammer, in welcher ein Zusatz, geeignet zur Anfbereitung von amplifizierten Nukeinsäuren für eine folgende Hybridisierung an Sonden - Oligonukleotide aufweisend
i) monovalente Kationen und
ii) ein Mg²⁺ Ionen -bindendes Agens,
lagerstabil als Trockenreagenz bevorratet ist,
wobei die zweite Kammer über eine Verbindung mit der ersten Kammer in Fluidkommunikation verbindbar ist, wobei die amplifizierten Nukleinsäuren mit dem Zusatz in Kontakt gebracht werden können, so dass eine Mischung aus Amplifikationsrohprodukt und Zusatz für eine Hybridisierungsreaktion zur Verfügung steht.

17. Anordnung nach Anspruch 16, wobei die monovalenten Kationen in Form von Na⁺ Ionen vorgesehen sind.

18. Anordnung nach einem der Ansprüche 16 oder 17, wobei das Mg²⁺ Ionen-bindende Agens EDTA ist.

19. Anordnung nach einem der Ansprüche 16 bis 18, wobei Mittel vorgesehen sind, um Fluid selektiv von der ersten in die zweite Kammer zu überführen.

20. Anordnung nach einem der Ansprüche 16 bis 18, wobei Mittel vorgesehen sind, um Fluid selektiv von der zweiten in die erste Kammer zu überführen.

21. Anordnung nach Anspruch 20, wobei Mittel vorgesehen sind, um ein Lösungsmittel in die zweite Kammer einzubringen.

22. Anordnung nach einem der Ansprüche 16 bis 21, ferner aufweisend eine Mikroarrayanordnung, welche auf einem Träger immobilisierte Sonden-Oligonukleotide aufweist.

23. Anordnung nach einem der Ansprüche 6 bis 22, ferner aufweisend Mittel zur optischen oder elektrochmischen Detektion hybridisierter Nukleinsäuren.

## Claims

1. Method for analyzing nucleic acids in a microfluidic device, comprising the following steps:
a) amplifying nucleic acids in a first chamber in the microfluidic device;
b) contacting the amplified nucleic acids with an additive suitable for conditioning amplified nucleic acids for a subsequent hybridization to probe oligonucleotides, comprising:
i) monovalent cations and
ii) an Mg²⁺ ion-binding agent,
wherein the additive is provided in a manner kept as a dry reagent in a second chamber in the microfluidic device; and
c) hybridizing the amplified nucleic acids to at least one probe oligonucleotide, wherein a mixture of amplification crude product and additive is used for the hybridization reaction.

2. Method according to Claim 1, wherein the monovalent cations are provided in the form of Na⁺ ions.

3. Method according to Claim 1 or 2, wherein the Mg²⁺ ion-binding agent is EDTA.

4. Method according to Claim 1, wherein, prior to contacting the additive with the amplified nucleic acids, a solvent is introduced into the second chamber.

5. Method according to Claim 4, wherein the additive is transferred in dissolved form from the second to the first chamber in order to contact the additive with the amplified nucleic acids.

6. Method according to any of Claims 1 to 3, wherein the amplified nucleic acids are transferred from the first to the second chamber in order to contact the additive with the amplified nucleic acids.

7. Method according to Claim 6, wherein the amplified nucleic acids in solution as amplification crude product are transferred to the second chamber and then conducted as a mixture with the additive to the at least one probe oligonucleotide.

8. Method according to any of the preceding claims, wherein the nucleic acids are amplified by means of PCR reaction.

9. Method according to any of the preceding claims, wherein the at least one probe oligonucleotide is immobilized in the form of a microarray arrangement on a carrier.

10. Method according to any of the preceding claims, wherein the amplified nucleic acids hybridized to the probe oligonucleotide are detected.

11. Method according to Claim 10, wherein the detection is effected using a label (a marking) of the amplified nucleic acids.

12. Method according to Claim 11, wherein the label is an optical label or an enzymatic label.

13. Method according to Claim 12, wherein the label catalyzes an enzymatic reaction which is optically or electrochemically detectable.

14. Method according to Claim 13, wherein the electrochemical detection involves a current measurement amplified by means of redox cycling.

15. Method according to any of the preceding claims, wherein the additive comprises a binder.

16. Arrangement for carrying out the method according to any of Claims 1 to 15, which is provided in a microfluidic device, comprising:
a) a first chamber, which is designed for the amplification of nucleic acids, and
b) a second chamber, in which an additive suitable for conditioning amplified nucleic acids for a subsequent hybridization to probe oligonucleotides, comprising
i) monovalent cations and
ii) an Mg²⁺ ion-binding agent,
is kept in storage-stable fashion as a dry reagent,
wherein the second chamber can be connected in fluid communication via a connection to the first chamber, wherein the amplified nucleic acids can be contacted with the additive, so that a mixture of amplification crude product and additive is available for a hybridization reaction.

17. Arrangement according to Claim 16, wherein the monovalent cations are provided in the form of Na⁺ ions.

18. Arrangement according to either of Claims 16 and 17, wherein the Mg²⁺ ion-binding agent is EDTA.

19. Arrangement according to any of Claims 16 to 18, wherein means are provided for transferring fluid selectively from the first to the second chamber.

20. Arrangement according to any of Claims 16 to 18, wherein means are provided for transferring fluid selectively from the second to the first chamber.

21. Arrangement according to Claim 20, wherein means are provided for introducing a solvent into the second chamber.

22. Arrangement according to any of Claims 16 to 21, furthermore comprising a microarray arrangement having probe oligonucleotides immobilized on a carrier.

23. Arrangement according to any of Claims 6 to 22, furthermore comprising means for optically or electrochemically detecting hybridized nucleic acids.

## Revendications

1. Procédé d'analyse d'acides nucléiques dans un dispositif microfluidique, comportant les stades suivants :
a) on amplifie des acides nucléiques dans une première chambre du dispositif microfluidique ;
b) on met les acides nucléiques amplifiés en contact avec un additif propre au traitement d'acides nucléiques amplifiés, pour une hybridation suivante sur des sondes-oligonucléotides et comportant :
i) des cations monovalents et
ii) un agent de fixation des ions Mg²⁺,
dans lequel l'additif est prévu en réserve en tant que réactif à sec dans une deuxième chambre du dispositif microfluidique ; et
c) on hybride les acides nucléiques amplifiés sur au moins une sonde-oligonucléotide en utilisant, pour la réaction d'hybridation, un mélange de produit brut d'amplification et d'additif.

2. Procédé suivant la revendication 1, dans lequel on prévoit les cations monovalents sous la forme d'ions Na⁺.

3. Procédé suivant la revendication 1 ou 2, dans lequel l'agent fixant les ions Mg²⁺ est l'EDTA.

4. Procédé suivant la revendication 1, dans lequel, avant de mettre l'additif en contact avec les acides nucléiques amplifiés, on introduit un solvant dans la deuxième chambre.

5. Procédé suivant la revendication 4, dans lequel on transvase l'additif sous forme dissoute de la deuxième à la première chambre, pour mettre l'additif en contact avec les acides nucléiques amplifiés.

6. Procédé suivant l'une des revendications 1 à 3, dans lequel on transvase les acides nucléiques amplifiés de la première à la deuxième chambre, pour mettre l'additif en contact avec les acides nucléiques amplifiés.

7. Procédé suivant la revendication 6, dans lequel on transvase les acides nucléiques amplifiés en solution sous la forme d'un produit brut d'amplification dans la deuxième chambre et on les envoie ensuite en mélange avec l'additif au au moins une sonde-olégonucléotide.

8. Procédé suivant l'une des revendications précédentes, dans lequel on effectue l'amplification des acides nucléiques au moyen d'une réaction PCR.

9. Procédé suivant l'une des revendications précédentes, dans lequel on immobilise au moins une sonde-oligonucléotide sous la forme d'un agencement en micromatrice sur un support.

10. Procédé suivant l'une des revendications précédentes, dans lequel on effectue une détection des acides nucléiques amplifiés hybridés.

11. Procédé suivant la revendication 10, dans lequel on effectue la détection en utilisant un label ( un marqueur ) des acides nucléiques amplifiés.

12. Procédé suivant la revendication 11, dans lequel le label est un label optique ou un label enzymatique.

13. Procédé suivant la revendication 12, dans lequel le label catalyse une réaction enzymatique, qui peut être détectée visuellement ou électrochimiquement.

14. Procédé suivant la revendication 13, dans lequel la détection électrochimique est une mesure de courant amplifié au moyen d'une cyclisation rédox.

15. Procédé suivant l'une des revendications précédentes, dans lequel l'additif comprend un liant.

16. Agencement pour la mise en oeuvre suivant l'une des revendications 1 à 15, qui est prévu dans un dispositif microfluidique, comprenant :
a) une première chambre, qui est conçue pour l'amplification d'acides nucléiques, et
b) une deuxième chambre, dans laquelle est mis en réserve, d'une manière stable au stockage en tant que réactif à sec, un additif propre au traitement d'acides nucléiques amplifiés pour une hybridation suivante sur des sondes-oligonucléotides et comprenant
i) des cations monovalents, et
ii) un agent fixant les ions Mg²⁺,
dans lequel la deuxième chambre peut communiquer avec la première chambre suivant une communication fluidique par une communication, les acides nucléiques amplifiés pouvant être mis en contact avec l'additif, de manière à disposer d'un mélange d'un produit brut d'amplification et d'additif, pour une réaction d'hybridation.

17. Agencement suivant la revendication 16, dans lequel les cations monovalents sont prévus sous la forme d'ions Na⁺.

18. Agencement suivant l'une des revendications 16 ou 17, dans lequel l'agent fixant les ions Mg²⁺ est l'EDTA.

19. Agencement suivant l'une des revendications 16 à 18, dans lequel il est prévu des moyens pour transvaser du fluide sélectivement de la première à la deuxième chambre.

20. Agencement suivant l'une des revendications 16 à 18, dans lequel il est prévu des moyens pour transvaser du fluide sélectivement de la deuxième à la première chambre.

21. Agencement suivant la revendication 20, dans lequel il est prévu des moyens pour introduire un solvant dans la deuxième chambre.

22. Agencement suivant l'une des revendications 16 à 21, comprenant, en outre, un agencement de micromatrice, qui comporte des sondes-oligonucléotides immobilisées sur un support.

23. Agencement suivant l'une des revendications 6 à 22, comprenant, en outre, des moyens de détection visuelle ou électrochimique d'acides nucléiques hybridés.
